# EUROPEAN PATENT APPLICATION

(11) **EP 4 202 954 A1**
(43) Date of publication of application: **28.06.2023**
(21) Application number: 21216858.7
(22) Date of filing: 22.12.2021
(51) Int. Cl.: G16H 40/67, G16H 80/00, H04W 84/04, H04W 12/03, H04W 4/90, H04W 12/088, H04W 12/121

(54) **REMOTE EMERGENCY CARE SYSTEM AND METHOD FOR VIDEO CONFERENCING**

(71) Applicant: Siemens Healthcare GmbH, 91052 Erlangen (DE)
(72) Inventor: Lautenschläger, Stefan, 90762 Nürnberg (DE); Cavallaro, Jonathan, East Melbourne 3002 (AU); Schmitt, Benjamin, Naremburn NSW 2065 (AU); Segaram, Lusan, Sydney 2154 (AU); Whiteside, Brooke, Noble Park North 3174 (AU); Whiteside, Christopher, Noble Park North 3152 (AU)

(57) **Abstract**

Remote emergency care system (1) for video conferencing between an emergency site (8) and a remote first communication device (2) of a physician, wherein the first communication device (2) comprises first videoconference equipment (17), the remote emergency care system (1) comprising:
- a second communication device (4) mounted to an mobile medical care center, wherein the second communication device (4) is adapted to communicate with the first communication device (2) via at least one first communication link (10, 10a, 10b, 10c, 10d) using at least one public mobile network (9, 9a, 9b, 9c, 9d),
- a mobile point of care terminal (7) as a third communication device (3) for medically trained staff, wherein the second and the third communication device (4, 3) communicate via a private, local second communication link (11) and wherein the third communication device (3) comprises second videoconference equipment (18), and
- a control device for establishing a video conference between the first and the third communication device (2, 3) using the first and second communication links (10, 10a, 10b, 10x, 10d, 11),
wherein the second communication link (11) is a private mobile communication link in a private mobile network (12) established by the second communication device (4), in particular a private LTE network.

## Description

The invention concerns a remote emergency care system for video conferencing between an emergency site and a remote first communication device of a physician, wherein the first communication device comprises first video conference equipment, the remote emergency care system comprising:
- a second communication device mounted to a mobile medical care center, wherein the second communication device is adapted to communicate with the first communication device via at least one first communication link using at least one public mobile network,
- a mobile point of care terminal as a third communication device for medically trained staff, wherein the second and the third communication device communicate via a private, local second communication link and wherein the third communication device comprises second video conference equipment, and
- a control device for establishing a video conference between the first and the third communication device using the first and second communication links.

The invention further concerns a method for operating such a remote emergency care system, in particular a method for video conferencing between a medical emergency site and a remote, stationary first communication device for a physician.

Medical emergency staff are usually paramedics, who are ideal all-rounders. Paramedics are trained in observing the emergency scene at a medical emergency site, that is, a pre-hospital or point-of-care setting, and initiate the most important actions to save a patient's life and transport them to a responsible, qualified hospital, in particular specialist.

In order to save time for diagnostic decisions and treatment initiation, but at the same time ensure a high level of diagnosis and treatment, expert knowledge is required at the emergency site, that is, in the pre-hospital setting. As an example, for this challenge, stroke is a very time-sensitive disease, as is reflected in the saying "time is brain". Shifting the diagnosis and treatment initiation of stroke to the pre-hospital setting, that is, the medical emergency site, makes utmost sense.

On the other hand, the symptoms of stroke are similar to those of other diseases, for example hypoglycaemia, making it very difficult or even impossible for a regular paramedic to decide whether the patient at the medical emergency site is having a stroke. Thus, the paramedic needs support from an experienced physician, in particular a neurologist.

It has thus been proposed to provide such remote assistance by a physician using a communication link, for example a dial-in audio link. However, since video information is essential in this case, audio-video-conferencing (shortly video conferencing in the following), is more expedient. Slightly simplified, this approach can be compared to a face time video chat using regular platforms known in the art, like Skype, Microsoft Teams or the like. However, high quality video needs to be transmitted, such that such video conferences are only possible if an adequate transmission band width can be assured.

In some proposals, the use of public mobile networks, that is, networks for public mobile telephony, which may also be called public cellular networks, has been suggested. Using a mobile point of care terminal, like, for example, a tablet or a mobile phone, the paramedic would call in to a remote first communication device, for example a desktop personal computer/work station or mobile remote terminal of a physician, running a corresponding application. However, as known from common experience, the standard public mobile network coverage varies significantly from location to location, and, in particular within buildings, there may even be a complete loss of the cellular signal. The essential part of the connection between the mobile point of care terminal and the first communication device is from the medical emergency site to the ambulance vehicle. One can assume that the ambulance vehicle itself may be parked on a street, where a proper connection to public mobile networks, in particular their base stations, should be available, in particular since ambulance vehicles often use special antennas optimized for reception and transmission, which may be mounted to the roof of the ambulance vehicle.

There have been several approaches to allow reliable video conferencing even from medical emergency sites with no access to public mobile networks, none of which has proven suitable for practical use. In a first approach, amplifying devices are used at the medical emergency site. Such amplifying devices typically use external power and external antennas to detect lower strength signals from, in particular, public mobile networks. This solution extends the range and signal strength, but not to the extent required/desired. An additional disadvantage is the necessity to carry additional equipment to the medical emergency site and that a power connection is required.

In another approach, the ambulance vehicle is equipped with WiFi antennas to create a wireless local area network (WLAN). That is, a second communication device in the ambulance vehicle provides a WiFi connection to the mobile point of care terminal as a third communication device. However, WiFi is using high frequency signals at limited power, such that the covered area is strongly reduced if there is no free line of sight between antenna and mobile terminal. These properties make WiFi a very suboptimal technology to be used in urban settings.

It is an object of the current invention to provide a reliable, secure, easily configurable way to enable video conferencing even in difficult environments at a medical emergency site.

This object is achieved by providing a remote emergency care system according to claim 1 and a method for video conferencing according to claim 11. Advantageous embodiments are described in the dependent claims.

In a remote emergency care system as initially described, according to the invention, the second communication link is a private mobile communication link in a private mobile network established by the second communication device, in particular a private LTE network.

Public mobile networks for mobile telephony, which may also be called public cellular networks, are usually defined by certain standards, in particular the LTE (Long Term Evolution) standard, which may be applied to the 4G technology as well as the 5G technology or even higher, future generations. Usually, such a public mobile network is established by a plurality of base stations, such that mobile terminals, for example mobile telephones, may communicate via these base stations. In particular, authentication means such as SIM cards may be used. For the 4G and 5G technologies, mobile telephony standards like LTE also allow private mobile networks to be established.

It is thus suggested to use such a local, private mobile network, in particular private LTE (for example in 4G, 5G, or even higher generation technology), to establish the second communication link from the second communication device to the third communication device, that is, from the mobile medical care center to the mobile point of care terminal at the medical emergency site. In particular, the second communication device in the mobile medical care center provides a mobile base station for the local, private mobile network, ensuring best coverage and signal strength up to, for example, one to two kilometres from this base station. In other words, the second communication device may comprise a base station for establishing the private mobile network providing the second communication link. The bandwidth of the private mobile network technology, for example 50 Mbit/s or more, the frequencies used and the signal strength in particular allow to penetrate various obstacles, in particular absorbing or damping structures like walls, easier in comparison to WiFi. The distance from the mobile medical care center at which video conferencing is reliably possible is massively increased, making the solution according to the invention practically feasible.

Inside the mobile medical care center, the data stream for the video conferencing using the combination of first and second communication links is forwarded to or from a router for establishing the first communication link, which may also be part of the second communication device. In particular, the second communication device may comprise a router for establishing the first communication link and the base station for establishing the private mobile network providing the second communication link, wherein the router and the base station are wiredly connected. Preferably, as known from the state of the art, an optimized antenna for the at least one public mobile network may be used.

The mobile medical care center may preferably be an ambulance vehicle, however, the invention may also be applied to other mobile medical care centers, for example emergency helicopters and/or mobile clinics. In particular, the medically trained staff may be emergency staff, for example comprising at least one paramedic.

The control device, which is in particular distributed, may comprise control units of each of the communication devices. Corresponding applications running at least on the first and third communication devices may be used to initiate video conferencing, wherein the control device establishes a video conferencing connection using the first and second communication links. The control device also controls data transfer during the video conference, as further explained below.

In summary, communication data of the established video conference to and from the remote first communication device of the physician, wherein in particular the physician is a medical expert for a condition to be assessed at the medical emergency site, is communicated via the combination of first and second communication links, wherein the second communication link is established in a private mobile network, in particular a private LTE network, satisfying all requirements for reliable and high-quality video conferencing at a medical emergency site. In particular, the video and audio signal may be transmitted to and received from even difficult emergency site locations, like, for example, basements, alleys, shopping centres and the like. The video transmission is reliable and communication ranges up to two hundred meters or more can be achieved even if absorbing or damping structures like walls exist. The second communication link can easily be made secure, is easily configurable and available with minimal set-up by the medically trained staff, in particular a paramedic. No additional configuration is required when moving from one emergency site to the next.

In summary, an ideal solution for all pre-hospital challenges/problems/questions that need expert input is provided. "Dial-in" experts are available whenever and wherever required. Deploying the remote emergency care system according to the invention increases the level of patient care and saves cost at the same time, as highly-paid resources, in particular physicians, are only consulted in cases where their knowledge is required, while they may still remain at a location remote to the medical emergency site, in particular a hospital/clinic, performing other tasks while not required for emergency care. This is large advantage, in particular compared to presence of the physician at the medical emergency site, when the expert, that is, the physician, is forced to be physically present in order to enable the same level of care as the technology according to the invention provides, even if his expertise is not required for the current case.

A special case where the current invention may be applied with particular advantage are so-called MSUs (Mobile Stroke Units). A mobile stroke unit is an ambulance especially equipped to diagnose, evaluate, and/or treat symptoms of an acute stroke. In addition to normal ambulance vehicles, an MSU may comprise a device for brain imaging, for example a computed tomography device, and/or a point-of-care laboratory. Additionally, using the remote emergency care system for video conferencing according to the invention, reliable telemedical interaction between the medically trained staff and the physician, in particular at a hospital, may be provided. In this manner, all the tools necessary for hyperacute assessment and treatment of stroke patients directly at the emergency site are provided. In particular, the MSU and its staff do not need to wait until the patient is brought to the ambulance vehicle (and, in particular, its device for brain imaging and/or point-of-care laboratory) to be diagnosed/assessed, but the assessment can be done faster at the medical emergency site itself. In particular in cases in which the patient is not having a stroke, the MSU and its staff can be released earlier from the current emergency, for example leaving the patient for normal ambulance care such that the next possible stroke emergency can be attended to.

In concrete embodiments, the router and/or the base station may comprise at least one component chosen from the group comprising a firewall, an intrusion detection and/or prevention system (IDS/IPS), and an encryption system. That is, in particular for the private mobile network, the respective equipment may provide built-in firewall functionality, intrusion detection and/or prevention, and/or full network encryption. For example, as the base station, a device called LXN500 available from Motorola Solutions, incorporated, may be used; and/or as the router, a Siemens RuggedCom RX1400 LTE may be employed.

But also generally, in preferred embodiments, the second communication link may be encrypted. In this manner, data security may be improved. Many solutions available for private mobile networks, in particular private LTE, already provide encryption means also suitable for the current invention.

For realizing the private mobile network, in particular the private LTE network, generally, two options exist. That is, the private mobile network may be based on WiMax technology or may use a licensed frequency spectrum. The WiMax unlicensed technology uses at least one unlicensed frequency spectrum to transmit signals in the private mobile network. Frequencies and/or bandwidths enabling a high range, making the technology far superior to WiFi, may be used, however, these frequencies may also be used by other operators, such that interferences may occur, which are not regulated via official entities. However, since a wide range of frequency spectra is available and private mobile networks are still quite rare, such radio frequency interferences may be sufficiently uncommon to still provide adequate reliability. In embodiments using the WiMax technology, a third communication device may comprise an internal or external WiMax modem. In embodiments, an external WiMax USB modem may be connected to the mobile point of care terminal.

To further reduce the risk of interference when using unlicensed frequency spectra, the remote emergency care system may comprise a frequency selection system for the second communication link, which is configured to the dynamically select a frequency spectrum which is not used at the current location of the mobile medical care center for the private mobile network. For example, a frequency scan may be performed to detect spectra in use and a frequency spectrum for the private mobile network may be dynamically chosen accordingly to further prevent interferences. Preferably, the frequency selection system may be provided in the mobile medical care center, in particular integrated into the second communication device, preferably into the base station.

On the other hand, a dedicated, licensed frequency spectrum may be used. This is a more expensive option, but enables interference protection in the radio frequency technology chosen. Another advantage of this option is that the mobile point of care terminal, which may be a mobile telephone, in particular a smartphone, and/or a tablet, does not require an additional modem if already equipped for LTE connectivity, which is typical for the mentioned mobile terminal examples.

In an especially preferred embodiment, the second communication device may be adapted to apply link aggregation to the multiple first communication links. Link aggregation refers to various methods of combining, that is aggregating, multiple network connections in parallel, such that the bandwidth can be increased beyond that of a single communication link and/or redundancy is provided, in particular if one of the first communication links fails or is not available. Link aggregation is also known as trunking, bundling, bonding, channelling or teaming. When using such a link aggregation or bonding technology, data to be streamed in a video conference may be split into multiple packages and sent via different first communication links, in particular using public mobile networks of different carriers (network providers). In other embodiments, it is also possible to use the same mobile communication network, but multiple authentication means, in particular SIM cards. Depending on the concrete configuration, link aggregation may multiply the amount of the data that can be exchanged within a given time period, add resiliency and/or provide redundancy. This is in particular important regarding the fact that the public mobile networks of different carrier often have different coverage in different regions, such that options that are actually available may be chosen for data transmission.

In other words, adding link aggregation (or bonding) technology for the at least one first communication link allows transferring larger amounts of data to and/or from a distant location, for example a hospital and/or a cloud server to which the first communication device is connected, much faster. This allows for faster evaluation, diagnosis and treatment initiation, but also allows the local clients to be "thin", as the resource-intense computing can be done centrally, thus reducing complexity and saving money. On the other hand, reliability is improved by providing redundancy.

Preferably, the second communication device, in particular the router, may comprise an authentication means, in particular a SIM card, for each first communication link. Such an authentication means may comprise a SIM card, such that, in particular, routers having multiple SIM card slots are preferred. As already indicated, in preferred embodiments, at least two of the first communication links may be provided by different public mobile networks, in particular public mobile networks established by a different carrier (mobile network provider). If all first communication links are provided in different public mobile networks, authentication means may be provided for each public mobile network to establish the respective first communication link.

In preferred embodiments, the second communication device may comprise an analysis unit for distributing the total bandwidth to the first communication links according to a maximum bandwidth information of the respective public mobile networks. Such an analysis unit may be placed between the base station and the router. Such an analysis unit may also be called bonding device. If, for example, two to four first communication links are used, in particular each by providing a respective authentication means, the bandwidth may be requested from the router by the analysis unit and the data to be transferred may be distributed accordingly to the first communication links.

Generally speaking, video conference equipment preferably comprises a camera, a microphone, a speaker and a display. While video conference equipment for medical emergency sites having no display has been proposed, it has been shown that the ability for the patient to actually see the remote expert, that is, the physician, increases compliance. While the first communication device may be a usual desktop personal computer/work station, where known video conference equipment, for example comprising a head set, the USB or integrated camera and a monitor as a display, may be used, it is also possible to use tablets and/or smartphones as first communication devices. Such tablets and/or smartphones, which are also preferred third communication devices, that is, mobile point of care terminals, may have all necessary video conference equipment already integrated. The first and the third communication device may run respective, corresponding applications as video conferencing software.

In especially preferred embodiments, the third communication device may be a tablet device comprising a controllably orientable camera, wherein the control device is configured to enable controlling the camera from the first communication device. Such dedicated tablet devices for medical emergency care have already been proposed, for example as telemedical tablets, and are preferably also employed in the context of the current invention. The possibility of remotely controlling the orientation of a camera of the third communication device allows the physician to directly choose which features they want to view, without any need for complicated instructions to the medically trained staff, in particular a paramedic.

The invention further concerns a method for video conferencing between a medical emergency site at a remote, stationary first communication device for a physician, wherein the first communication device comprises first video conference equipment, using a remote emergency care system comprising:
- a second communication device mounted to a mobile medical care center, wherein the second communication device is adapted to communicate with the first communication device via at least one first communication link using at least one public mobile network,
- a mobile point of care terminal as a third communication device for medically trained staff, wherein the second and the third communication device communicate via a private, local second communication link and wherein the third communication device comprises second video conference equipment, and
- a control device for establishing a video conference between the first and the second communication device using the first and second communication links,
- wherein, according to the invention, a private mobile communication link in a private mobile network established by the second communication device, in particular a private LTE network, is used as the second communication link.

All features and remarks regarding the remote emergency care system according to the invention correspondingly apply to the method according to the invention, such that the same advantages can be achieved.

Other objects and features of the present invention will become apparent from the following detailed description considered in conjunction with the accompanying drawings. The drawings, however, are only principle sketches designed solely for the purpose of illustration and do not limit the invention. The drawings show:
Fig. 1 a remote emergency care system according to the invention, and
Fig. 2 a detailed view of a second communication device.

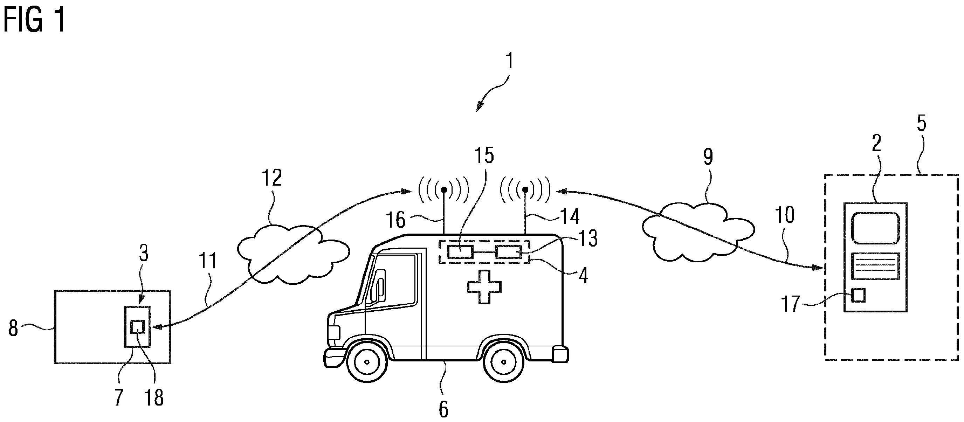

Fig. 1 illustrates the use of a remote emergency care system 1 according to the present invention. The remote emergency care system 1 is configured to provide video conferencing for telemedicine between a remote first communication device 2, which may be part of the system 1, of a physician (expert) and a third communication device 3 via a second communication device 4. The first communication device 2 may, for example, the associated with at least one physician in a hospital 5. The second communication device 4 is mounted to an ambulance vehicle 6 serving as a mobile medical care center, which, in this case, may be a mobile stroke unit (MSU). The third, mobile communication device 3 is a mobile point of care terminal 7, in this case a tablet device, which is carried along by medically trained staff, in particular to an emergency site 8, which may also be called pre-hospital setting or emergency point of care. The medical emergency site 8 may well be a location, for example in a basement, a shopping mall or the like, where public mobile networks 9 may not be available. The ambulance vehicle 6, on the other hand, is typically parked outside, where there is good reception regarding the public mobile networks 9. Hence, communication of the first communication device 2 and the third communication device 3 for video conferencing is established via two separate communication links 10, 11, namely at least one first communication link 10 using the at least one public mobile network 9 (public cellular network) and a second communication link 11 using a private mobile network 12 established by the second communication device 4 in the ambulance vehicle 6. In this embodiment, the local, private mobile network 12 is a private LTE network. Since the third communication device 3, which, in this case, is a tablet, in particular a telemedicine tablet, is already equipped for LTE communication, no modification of the third communication device 3 is necessary.

To communicate in the at least one public mobile network 9, the second communication device 4 may comprise a router 13 and an optimized antenna 14 mounted to the roof of the ambulance vehicle 6. The router 13 is wirelessly connected to a base station 15 which may comprise its own associated antenna 16, also mounted to the roof of the ambulance vehicle 6, wherein the base station 15 establishes the local, private mobile network 12.

The first communication device 2 and the third communication device 3 each comprise video conference equipment 17, 18, in this case a microphone, a speaker, a camera, and a display. The first and the third communication devices 2, 3 additionally each run a video conferencing application. Control units of each of the communication devices 2, 3 and 4 together form a distributed control device of the remote emergency care system 1, which is adapted to establish a video conference between the first communication device 2 and the third communication device 3 via the second communication device 4 using the at least one first communication link 10 and the second communication link 11. The control device also controls data transfer once the video conferencing connection is established.

If, for example, a paramedic of the emergency staff needs expert assistance at the medical emergence site 8, for example to assess a patient regarding a possible stroke, they use the application on the third communication device 3 to request a video conference to a physician at the first communication device 2. If not already established, the second communication link 11 is created in the private mobile network 12, and the at least one first communication link 10 is established. The video conference application at the remote fist communication device 2 outputs a notification of an incoming call, and, once accepted by the physician, the video conference is started, wherein the video conference data is streamed using the at least one first and second communication links 10, 11, controlled by the control device. Preferably, the camera of the video conference equipment 18 may be controllably orientable from the remote first communication device 2. As the third communication device 3 also comprises a display, the patient is able to see the physician, thus increasing compliance, while the physician may appropriately choose the field of view of the camera of the video conference equipment 18. The physician may then assess the condition of the patient, in particular regarding a possible stroke. If the patient is diagnosed as having no stroke, if the ambulance vehicle 6 is an MSU, the emergency site 8 may be cleared for a normal ambulance to take care of the patient.

It is noted that, generally, encryption may be used in the local, private mobile network 12, such that the second communication link 11 is encrypted. Encryption may, of course, also be applied to the at least one first communication link, as known. Each and/or at least one of the router 13 and the base station 15 may comprise a firewall, an intrusion detection and/or prevention system, and/or an encryption system.

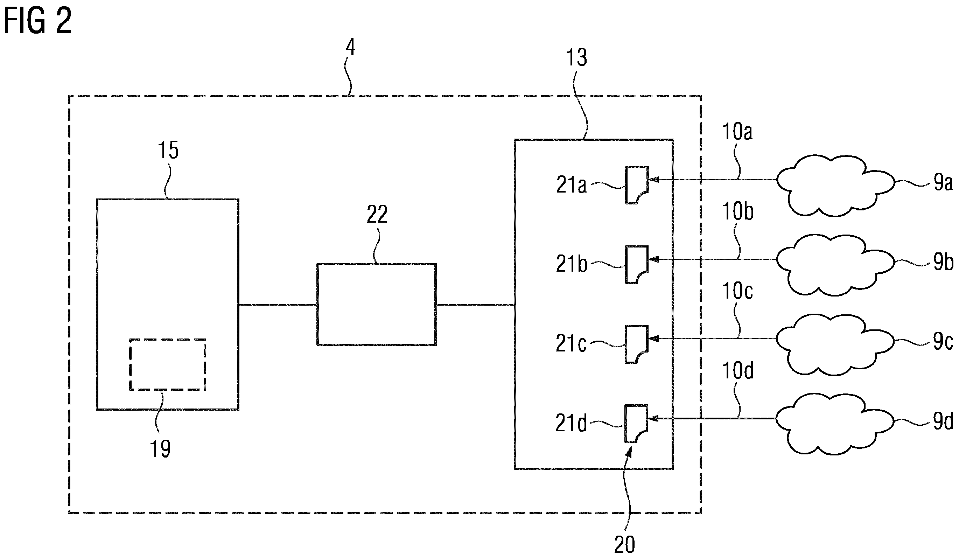

The private mobile network 12 may use a licensed frequency spectrum, however, embodiments are also conceivable in which WiMax technology is used, that is, unlicensed frequency spectra are employed. In this case, as indicated in fig. 2, which is a schematic drawing of the second communication device 4, the base station 15 may optionally comprise a frequency selection system 19, which scans frequencies at each emergency area and chooses a frequency spectrum not in use in the emergency area for establishing the private mobile network 12.

It is generally noted that the private mobile network 12 may have a range of one to two kilometres and/or provide a bandwidth of 50 Mbit/s to one Gbit/s. In this example, 4G technology is used, however, it is also possible to use 5G technology or higher generations, such that even higher bandwidths are possible.

As the at least one first communication link 10 uses at least one public mobile network, the bandwidth may, in some cases, be lower and/or signals strength may be poor and/or public networks 9 may even not be available at a current location of the ambulance vehicle 6, despite the optimized antenna 14. Hence, in the preferred embodiment shown, link aggregation regarding the communication between the first communication device 2 and the second communication device 4 is applied.

In this case, a router 13, as shown in fig. 2, having multiple authentication means 20, in this case SIM cards 21a, 21b, 21c and 21d, in respective SIM card slots is used, wherein, in the shown embodiment, each SIM card 21a to 21d provides first communication links 10a, 10b, 10c and 10d in public mobile networks 9a, 9b, 9c, 9d of different carriers, such that, if one or more of the public mobile networks 9a to 9d is not available, redundancy is provided. Additionally, as already described, link aggregation is applied to the first communication links 10a to 10d in the sense that the data stream of the video conference is distributed to these first communication links 10a to 10d according to the bandwidth currently provided. To this end, an analysis unit 22 (bonding device) is provided interposed between the base station 15 and the router 13. The analysis unit 22 requests currently available bandwidths for each of the communication links 10a to 10d and correspondingly distributes data in multiple packets to these first communication links 10a to 10d.

Due to the so-provided high bandwidth in the private mobile network 12 and due to link aggregation in the public mobile network 9a to 9d, a video conference of high quality and reliability is established, in particular providing high definition or even better video.

Although the present invention has been described in detail with reference to the preferred embodiment, the present invention is not limited by the disclosed examples from which the skilled person is able to derive other variations without departing from the scope of the invention.

## Claims

1. Remote emergency care system (1) for video conferencing between an emergency site (8) and a remote first communication device (2) of a physician, wherein the first communication device (2) comprises first videoconference equipment (17), the remote emergency care system (1) comprising:
- a second communication device (4) mounted to an mobile medical care center, wherein the second communication device (4) is adapted to communicate with the first communication device (2) via at least one first communication link (10, 10a, 10b, 10c, 10d) using at least one public mobile network (9, 9a, 9b, 9c, 9d),
- a mobile point of care terminal (7) as a third communication device (3) for medically trained staff, wherein the second and the third communication device (4, 3) communicate via a private, local second communication link (11) and wherein the third communication device (3) comprises second videoconference equipment (18), and
- a control device for establishing a video conference between the first and the third communication device (2, 3) using the first and second communication links (10, 10a, 10b, 10x, 10d, 11),
**characterized in that** the second communication link (11) is a private mobile communication link in a private mobile network (12) established by the second communication device (4), in particular a private LTE network.

2. Remote emergency care system (1) according to claim 1, **characterized in that** the second communication device (4) comprises a router (13) for establishing the first communication link (10, 10a, 10b, 10c, 10d) and a base station (15) for establishing the private mobile network (12) providing the second communication link (11), wherein the router (13) and the base station (15) are wiredly connected.

3. Remote emergency care system (1) according to claim 2, **characterized in that** that the router (13) and/or the base station (15) comprise at least one component chosen from the group comprising a firewall, an intrusion detection and/or prevention system and/or an encryption system.

4. Remote emergency care system (1) according to one of the preceding claims, **characterized in that** the second communication link (11) is encrypted.

5. Remote emergency care system (1) according to one of the preceding claims, **characterized in that** the private mobile network (12) is based on WiMax technology or uses a licensed frequency spectrum.

6. Remote emergency care system (1) according to one of the preceding claims, **characterized in that** it comprises a frequency selection system (19) for the second communication link (11), which is configured to dynamically select a frequency spectrum which is not used at the current location of the mobile medical care center for the private mobile network (12) .

7. Remote emergency care system (1) according to one of the preceding claims, **characterized in that** the second communication device (4) is adapted to apply link aggregation to the multiple first communication links (10, 10a, 10b, 10c, 10d).

8. Remote emergency care system (1) according to claim 7, **characterized in that** the second communication device (4), in particular the router (13), comprises authentication means (20), in particular a SIM card (21a, 21b, 21c, 21d), for establishing the first communication links (10, 10a, 10b, 10c, 10d) .

9. Remote emergency care system (1) according to claim 7 or 8, **characterized in that** the second communication device (4) comprises an analysis unit (22) for distributing the total bandwidth to the first communication links (10, 10a, 10b, 10c, 10d) according to a maximum bandwidth information of the respective public mobile networks (9, 9a, 9b, 9c, 9d).

10. Remote emergency care system (1) according to one of the preceding claims, **characterized in that** the third communication device (3) is a tablet device and comprises a controllably orientable camera, wherein the control device is configured to enable controlling the camera from the first communication device (2).

11. Method for video conferencing between an emergency site (8) and a remote first communication device (2) for a physician, wherein the first communication device (2) comprises first videoconference equipment, using a remote emergency care system (1) comprising:
- a second communication device (4) mounted to an mobile medical care center, wherein the second communication device (4) is adapted to communicate with the first communication device (2) via at least one first communication link (10, 10a, 10b, 10c, 10d) using at least one public mobile network (9, 9a, 9b, 9c, 9d),
- a mobile point of care terminal (7) as a third communication device (3) for medically trained staff, wherein the second and the third communication device (4, 3) communicate via a private, local second communication link (11) and wherein the third communication device (3) comprises second videoconference equipment (18), and
- a control device for establishing a video conference between the first and the third communication device (2, 3) using the first and second communication links (10, 10a, 10b, 10x, 10d, 11),
**characterized in that** a private mobile communication link in a private mobile network (12) established by the second communication device (4), in particular a private LTE network, is used as the second communication link (11).
